# EUROPÄISCHE PATENTSCHRIFT

(11) **EP 1 567 206 B1**
(45) Veröffentlichungstag und Bekanntmachung des Hinweises auf die Patenterteilung: **17.02.2010**
(21) Anmeldenummer: 03779539.0
(22) Anmeldetag: 05.12.2003
(51) Int. Cl.: A61L 31/14, A61L 27/56

(54) **GEZÜCHTETE KNORPELZELLEN BEINHALTENDES IMPLANTAT UND VERFAHREN ZUR HERSTELLUNG**
IMPLANT CONTAINING CULTURED CARTILAGE CELLS AND METHOD FOR PRODUCING THIS IMPLANT
IMPLANT CONTENANT DES CELLULES CARTILAGINEUSES ET PERMETTANT DE LES FAIRE CROITRE, ET PROCEDE DE PRODUCTION DE CET IMPLANT

(30) Priorität: 05.12.2002 AT 18252002
(43) Veröffentlichungstag der Anmeldung: 31.08.2005
(73) Patentinhaber: WINKLER, Heinz, A-1232 Wien (AT)
(72) Erfinder: WINKLER, Heinz, A-1232 Wien (AT)
(74) Vertreter: Wildhack & Jellinek
(86) Internationale Anmeldenummer: PCT/AT2003/000362
(87) Internationale Veröffentlichungsnummer: WO 2004/050135

(56) Entgegenhaltungen:
- US-A- 4 553 272
- US-B1- 6 187 329

## Beschreibung

Die Erfindung betrifft ein neues Implantat bzw. derartigen Implantatkörper für die Regeneration von Gelenksdefekten im menschlichen oder tierischen Körper, welches bzw. welcher mit einem Trägerkörper (1) aus einem körperverträglichen, Poren-, Zell- bzw. Schwammstruktur aufweisenden Material, insbesondere aus einem spongiösen Knochen menschlicher oder tierischer Herkunft, gebildet ist und zumindest teilweise mit einer Suspension von Gewebezellen tränkbar bzw. getränkt ist, ein Verfahren zur Herstellung des neuen Implantates sowie dessen Verwendung für den oben genannten Zweck.

Es ist bekannt, Knorpelgewebe zur Regeneration von Gelenksdefekten aus autologen Zellen zu züchten. Diese können durch Biopsie aus gesunden Knorpelabschnitten entnommen, aber auch aus mesenchymalen Stammzellen, beispielsweise aus dem Knochenmark, differenziert werden. Die Züchtung erfolgt in der Regel in einem flüssigen Nährmedium. Die Implantation wird üblicherweise in Form der Zuführung einer Zellsuspension durchgeführt, was beträchtliche technische Probleme, aber auch einen Verlust von Knorpelzellen und eine Verschlechterung des Ergebnisses mit sich bringt.

Die vorliegende Erfindung hat sich die Aufgabe gestellt, ein Implantat zu schaffen, welches einem natürlichen Gewebe möglichst ähnliche mechanische Eigenschaften aufweist und eine gute Verbindung mit dem umliegenden Gewebe, insbesondere dem subchondealen, also unter dem Knorpel gelegenen Knochen, sicherstellt.

Zur Lösung dieser Aufgabe schlägt die Erfindung den Einsatz eines Trägerkörpers aus einem körperverträglichen, poren- oder schwammartigen Struktur aufweisenden Material, insbesondere aus einem spongiösen Knochen menschlicher oder tierischer Herkunft, vor, der zumindest teilweise mit einer Knorpelzellen-Suspension getränkt ist.

Gegenstand der Erfindung ist somit ein neuartiges Implantat bzw. Transplantat bzw. ein neuer Implantat- bzw. Transplantatkörper der eingangs genannten Art, welcher dadurch gekennzeichnet ist, dass es bzw. er mit einem Trägerkörper mit einem zumindest von einem Teilbereich seiner Oberfläche (2) ausgehenden, innerhalb desselben endenden Kanälen (3) für die Infiltration der jeweils für die Regeneration vorgesehenen Gewebs-Zellsuspension versehen ist.

Gemäß **Anspruch 2** ist eine Tränkung des Trägerkörpers mit Knorpelgewebe besonders bevorzugt.

Ein derartiger Trägerkörper hat den Vorteil, dass er leicht zu beschaffen ist und eine dreidimensionale Struktur aufweist, die ideale morphologische Bedingungen für eine Ansiedlung von Zellen bietet. Mit Hilfe eines derartig präparierten Trägerkörpers kann das Im- bzw. Transplantat exakt an den Ort der zu behandelnden Defekte im Gelenksbereich implantiert werden und auch sicher gegen Lageveränderungen bzw. Zellverluste fixiert werden.

Um eine optimale Tränkung des Trägerkörpers mit der Zellen-Suspension sicherzustellen, ist eben jener Bereich, von dem aus die defekt-heilende Wirkung ausgehen soll, also ein entsprechender Teilbereich der Oberfläche des Trägerkörpers mit von dort ausgehenden Kanälen für die Infiltration der Zellensuspension versehen.

An dieser Stelle soll nun kurz auf den Stand der Technik auf diesem Gebiet der Implantate bzw. Transplantate eingegangen werden:

So ist aus der DE 40 40 872 A1 ein im Dentalbereich implantierbarer Gewebeträger bekannt, wobei zur Regeneration des Desmodont auf demselben künstlich vermehrte bindegewebsartige Zellen aufgebracht sind, die Desmodont-Charakter haben. Als Gewebeträger kommt dort ein enossales Implantat, ein flächiger, insbesondere membranartiger Träger, Knochen oder ein Knochenersatzmaterial oder eine Kombination solcher Materialien zum Einsatz. Die aufgebrachten Zellen stammen aus patienteneigenem Gewebe, Spendergewebe oder einer eigens gezüchteten Zelllinie, wobei es sich um Desmodontalzellen oder Zellen handeln kann, welche sich nach Implantation des Gewebeträgers zu Gewebe mit Desmodont-Charakter differenzieren.

Über Probleme der Einbringung der entsprechenden Zellsuspension, welche jedoch, wie bekannt ist, in der Praxis immer wieder auftreten, wird dort nicht berichtet und somit auch nicht über eventuelle Ansätze zur Lösung solcher Probleme.

Im wesentlichen gilt dies auch für das Verfahren zur Präparierung von zur Verpflanzung geeigneten Knochen gemäß der DE 961 654 A, wobei dort die Tränkung des Knochengewebes mit Zellen nicht im Vordergrund steht, sondern vielmehr die Präparierung von Knochenmaterial unter Entfernung der Weichgewebe desselben innen und außen, der Fettsubstanzen und des Blutfarbstoffes, wobei so vorgegangen wird, dass die Knochen mit Eiweißentfernungsmitteln, wie z.B. mindestens 10%iger H2O2-Lösung oder Trypsinlösung behandelt werden.

Die DE 41 21 043 A1 betrifft ein Knochenersatzmaterial, das in einer porösen Matrix ein oder mehrere Polypeptid(e) mit der biologischen Wirkung von Fibroblasten-Wachstumsfaktoren enthält. Das dort beschriebene Einheilungsverhalten entspricht dem von autologem Knochentransplantat.

Die oben angeführten Probleme mit der gleichartigen Einbringung von Zellmaterial in des Knochenersatzmaterial und Wege zur Lösung derselben sind dort ebenfalls nicht angesprochen.

Schließlich betrifft die US 4,553,272 ein Reparaturmaterial für menschliches Gewebe auf Implantat-Basis, wobei eine porenartige bzw. zelluläre, innere Struktur dieses Materials vorgeschlagen wird, wo von der Oberfläche her die Größe der Poren bzw. Zellen her zunimmt. Auch diese US-A schlägt keine der vorliegenden Erfindung nahekommende Lösung für die Problematik der Tränkung eines zelligen, porösen oder schwammartigen Materials vor.

Die in die neuen Implantate eingetieften Infiltrier-Kanäle haben bevorzugter Weise einen Durchmesser von etwa 300 bis 500 µm, wie dem **Anspruch 3** zu entnehmen ist. Sie können auf einfache Weise mechanisch mittels Bohren oder Fräsen, mittels Laserstrahlen oder mittels Hochdruck-Wasserstrahlen hergestellt werden.

Vorteilhaft ist es, wenn der Trägerkörper lediglich in einem Teilbereich, nämlich dort, wo der Knorpel gebildet bzw. wiederhergestellt werden soll, mit der Zellen-, vorzugsweise Knorpelzellen-Suspension getränkt ist.

Wie dem **Anspruch 3** zu entnehmen, ist die spitz-konusartige Form der Infiltrier-Kanäle im Implantat-Körper besonders bevorzugt. Durch diese Formgebung wird, wie gefunden wurde, erreicht, dass es beim Infiltrieren des Trägerkörpers mit der Knorpelgewebszellen-Suspension im oberflächennahen Bereich desselben zu einer wesentlich intensiveren Imprägnierung des schwammartigen Knochenmaterials kommt, als im Bereich der verjüngten unteren Enden der Infiltrier-Kanäle. Infolge der konusartigen Ausbildung dieser Kanäle ist ein kontinuierlicher Übergang der Intensität der Tränkung mit der Suspension gewährleistet, und damit wird erreicht, dass im Bereich der Oberfläche wesentlich mehr Knorpelgewebe gebildet wird, als im Bereich der Kanal-Enden, dass also ein abnehmender kontinuierlicher Gradient an Knorpelgewebs-Anteil im Implantat zu dessen Inneren hin eintritt und somit ein kontinuierlicher Übergang von "überwiegend Knorpelgewebe" zu "überwiegend Knochengewebe" erreicht wird, was den natürlichen Verhältnissen entspricht bzw. denselben besonders nahe kommt.

Die spitz-konusartige Ausbildung der Infiltrier-Kanäle hat weiters den Vorteil, dass bei der im Folgenden beschriebenen, weiters besonders bevorzugten Ausführungsform der Erfindung, welche in einer gezielten und im Rahmen der Erfindung besonders vorteilhaften Teil-Demineralisierung des Knochengewebes besteht, das Knochenmaterial in Nähe der Oberfläche stärker demineralisiert wird als in der Tiefe, was ebenfalls dazu führt, dass im Oberflächen-Bereich des Implantat-Körpers ein stärkerer Abbau von Knochen-Hartmaterial erfolgt, als in der Tiefe der Infiltrierkanäle und somit der Trägerkörper im Bereich der Oberfläche "knochenartiger" ist als in der Tiefe, wobei auch hierbei die "Knorpelartigkeit" kontinuierlich zum Trägerkörper-Inneren hin abnimmt und die Knochenähnlichkeit entsprechend zunimmt, was den realen Verhältnissen in Gelenken entspricht.

Dies gilt in gleichem Maße für die später noch beschriebenen Infiltration von Nährlösungen und. dgl.

Die Tiefe der Infiltrier-Kanäle erstreckt sich lediglich über einen Teil der Dicke bzw. Materialstärke des Trägerkörpers, siehe dazu **Anspruch 4**. Besonders dann, wenn sich die Infiltrierkanäle in Richtung zum Trägerkörper-Inneren verjüngen, ist der Vorteil gegeben, dass eine Art gleitender Übergang von knorpeligen in knöcherne Anteile des Implantats sichergestellt ist.

Gemäß einer weiteren, aus dem **Anspruch 5** hervorgehenden, bevorzugten Ausführungsform der Erfindung hat der Trägerkörper bzw. Implantatkörper Zylinder-Gestalt und die Infiltrier-Kanäle gehen bevorzugt von dessen Deck- bzw. Grundfläche bzw. von einer seiner Endflächen aus.

Gemäß **Anspruch 6** ist vorgesehen, dass die die Infiltrier-Kanäle aufweisende Deck- oder Grundfläche des Implantat-Zylinders konvex ausgebildet ist. Dadurch ist eine einfache und optimale Verankerung desselben in entsprechender zylindrischen Ausnehmungen des Knochens möglich, und die jeweils den geometrischen Verhältnissen im zu regenerierenden Gelenk entsprechende konvexe Stirnseite entspricht so der Form des Gelenkes optimal und erlaubt auf diese Art ein rasches Einheilen. Es soll selbstverständlich darauf verwiesen werden, dass die Gestalt des Trägerkörpers auch jede andere Form bzw. Topographie des zu behandelnden Defekts angepasst werden kann.

Um die Bedingungen für das Knorpelwachstum zu verbessern, ist es weiters von besonderem Vorteil, und stellt eine besonders bevorzugte Ausführungsvariante der Erfindung dar, wenn jedenfalls der mit der (Knorpel-)Zellensuspension zu tränkenden bzw. getränkte Bereich des Implantatkörpers zumindest teilweise demineralisiert ist, wie dem **Anspruch 7** zu entnehmen, wobei insbesondere im Falle von Spitzkonus-Infiltrierkanälen der Vorteil der gradientenartig in die Tiefe abnehmenden Demineralisierung gegeben ist.

Was das einen weiteren wesentlichen Gegenstand der Erfindung bildende Verfahren zur Herstellung des wie oben in seiner Grundkonzeption und in verschiedenen Ausführungsvarianten beschriebenen Implantates bevorzugte Verfahren zur Herstellung des Implantates betrifft, so ist dasselbe im **Anspruch 8** näher beschrieben.

Die Infiltrier-Kanäle begünstigen das Eindringen der Suspension in die zellige Poren- bzw. Schwammstruktur des Trägerkörpers und gewährleisten eine vollständige Tränkung im jeweils dafür vorgesehenen Teilbereich des Implantatkörpers.

Das Einbringen der Zellen-Suspension kann bevorzugter Weise zusätzlich dadurch unterstützt werden, dass nach dem Eintauchen des Trägerkörpers mit den mittels Bohren Laserstrahl oder Druckwasserstrahl eingetieften Infiltrier-kanalen in die Zellen-Suspension an den Trägerkörper Unterdruck oder ein Vakuum angelegt wird, wie im **Anspruch 9** geoffenbart.

Vorteilhaft wird - siehe dazu den **Anspruch 10** - zumindest der zu tränkende Bereich des Trägerkörpers vor dem Tränken einem Reinigungs- und/oder Demineralisierungsprozess unterzogen. Mittels des Reinigungsprozesses werden Markbestandteile, wie Fette, verschiedene sonstige Zellen, Bindegewebe, Gefäße od.dgl., entfernt, wodurch die Anzahl der Poren und Hohlräume vermehrt wird, in welchen sich z.B. unter Zusatz entsprechender Nährmedien die mit der Zellen-Suspension eingebrachten Zellen, insbesondere Knorpelzellen, ansiedeln können.

Außerdem ist der Vorteil gegeben, dass mittels der Demineralisierung die mechanischen Eigenschaften des Trägers "knorpelähnlicher" werden und sich damit auch die Bedingungen für das Knorpelzellwachstum verbessern.

Um die Demineralisierung vorzunehmen, wird gemäß einer vorteilhaften Variante des **Anspruches 10** zumindest der zu tränkende Bereich des Trägerkörpers in ein Demineralisierungs-Medium, wie beispielsweise Salzsäure, insbesondere in einer Konzentration von um die 0,5 Mol/L, eingebracht oder eingetaucht, worauf bevorzugterweise an den Trägerkörper Unterdruck bzw. ein Vakuum angelegt wird, wodurch ein besonders komplettes Tränken desselben mit der Zellen-Suspersion sichergestellt ist. Danach werden das Demineralisierungs-Medium und die darin enthaltenen, aus dem Knochen herausgelösten Salze durch Spülen entfernt.

Um die Haftung im Trägerkörper bzw. das Wachstum der jeweiligen Zellen, insbesondere Knorpelzellen, zu begünstigen, kann erfindungsgemäß der zu tränkende Bereich des Trägerkörpers mit einem Nährmedium, beispielsweise mit Hyaluronsäure oder Kollagen, imprägniert werden, wie im **Anspruch 11** geoffenbart. Dabei kann die Imprägnierung vor der Tränkung des Trägerkörpers mit der (Knorpel-)Zellen-Suspension erfolgen.

Es kann alternativ vorgesehen sein, das Tränken des Trägerkörpers mit einem Gemisch aus einer Zellen- bzw. Knorpelzellen-Suspension und einem die Bildung und Vermehrung der Zellen begünstigendem Medium, wie beispielsweise Hyaluronsäure oder Kollagen, gleichzeitig vorzunehmen.

In der die Erfindung erläuternden Zeichnung ist ein erfindungsgemäßes Implantat schematisch dargestellt. Dieses basiert auf einem Trägerkörper 1, der im gezeigten Fall aus spongiösen Knochen menschlicher oder tierischer Herkunft besteht. Der Trägerkörper weist hier Zylinder-Gestalt auf, wobei die End- bzw. Deckfläche 2 dieses zylinderförmigen Trägerkörpers 1 konvex gewölbt ausgebildet ist. Von dieser konvexen Endfläche 2 erstrecken sich - hier parallel zueinander ausgerichtete, voneinander jeweils um mehr als ihre Durchmesser-Dimension beabstandete Infiltrier-Kanäle 3 in Richtung zum Inneren des Trägerkörpers 1 hin, die, von der konvexen Deckfläche 2 des Trägerkörpers 1 ausgehend, sich in Richtung zum Trägerkörper-Inneren hin spitzkonusartig verjüngen. Der Mitten-Durchmesser dm der Kanäle 3 kann zwischen etwa 300 und 500 µm betragen, der Abstand der Kanäle 3 voneinander beträgt beispielsweise etwa 1 bis 3 mm. Die Tiefe der Kanäle 3 ist so gewählt, dass am Implantationsort eine jeweils gewünschte Knorpeldicke entsteht.

Die Herstellung der Kanäle 3 kann mittels eines mechanischen Bohrers erfolgen, aber auch mittels Laserstrahl- bzw. Wasserstrahlbohrens.

Nach dem Herstellen der Infiltrier-Kanäle erfolgt eine Reinigung und Demineralisierung des aus einem spongiösen Knochenmaterial bestehenden Trägerkörpers, insbesondere mit Salzsäure, 0,5 mol/l.

Die Reinigung erfolgt mittels einer Serie von Bädern, bevorzugt in Fettentzugs-Lösungen, wodurch Fette, verschiedene Zellen, Bindegewebe und Gefäße aus dem Knochenmaterial entfernt werden, sodass zusätzliche Hohlräume für die Besiedelung durch die zu infiltrierenden (Knorpel-)Zellen entstehen.

Für diese Deminerallsierung wird der Trägerkörper 1 mit seinem die Kanäle 3 aufweisenden Endbereich in ein Bad mit Salzsäure eingebracht, und es wird an den Trägerkörper 1 Vakuum angelegt, wodurch das Eindringen der Salzsäure-Lösung in den die Zell-, Poren- bzw. Schwammstruktur des Trägerkörpers unterstützt wird. Sowohl das Vakuum als auch die Dauer der Demineralisierung werden so gewählt, dass eine optimale, knorpelähnliche Konsistenz des Knochens erzielt wird.

Anschließend kann der so vorbehandelte Trägerkörper 1 mit einem die Bildung von Knorpelzellen begünstigenden Nährmedium, beispielsweise mit Hyaluronsäure oder Kollagen, imprägniert werden.

Nach dieser Vorbehandlung erfolgt ein Tränken des Trägerkörpers 1 mit der (Knorpel-)Zellen-Suspension. Hierfür wird jener Bereich des Trägerkörpers, in welchem die Bildung von Knorpelgewebe erfolgen soll, in eine an sich übliche Knorpelzellen-Suspension eingetaucht, welche vorher durch Biopsie entnommene Knorpelzellen enthält. Bei Einsatz entsprechender Nährmedien können auch mesenchymale Stammzellen, beispielsweise aus dem Knochenmark oder aus Nabelschnurblut, verwendet werden.

Durch Anwenden von Unterdruck bzw. Vakuum wird das Eindringen der Zellen-Suspension entlang der Infiltrier-Kanäle in die Trabekel-Zwischenräume gewährleistet. Alternativ kann auch gleich die Zellen-Suspension mit dem jeweils gewählten Nährmedium vermischt werden und das Gemisch kann, unterstützt durch Vakuum in den Trägerkörper eingebracht werden.

Anschließend wird das Aufsaugen der Suspension in die Schwammstruktur des Trägerkörpers und dem Anhaften an den Oberflächen der Poren abgewartet. Durch das Nährmedium wird in der Folge eine weitere Ausbreitung der Zellen sichergestellt bzw. die Bildung und Vermehrung der Knorpel-Grundsubstanz angeregt. Sobald der gewünschte Abschnitt des Trägerkörpers mit Knorpelgewebe ausgefüllt ist, ist die Präparation abgeschlossen.

Im Zuge der Besiedelung kann durch Beifügung von Collagenagen bzw. sonstigen lytischen Enzymen die Trabekelstruktur schrittweise aufgelöst werden, sodass letztlich Raum für einen vollständigen Ersatz des Trägerkörpers durch die von den Zellen gebildete Knorpelmatrix geschaffen wird.

Das derart erhaltene Implantat kann in der Folge in einem defekten Gelenksbereich implantiert werden, wobei zunächst in den für den Einsatz des Implantates vorgesehenen Knochen zylinderförmige Ausnehmungen eingearbeitet werden, in welche ein entsprechender Implantat-Körpers mit Passsitz eingesetzt wird. Durch entsprechende Dimensionierung des Trägerkörper-Zylinders im Knochen (etwa 0,5 bis 1 mm geringerer Durchmesser) wird ein Passsitz mit dem knöchernen Anteil des Implantats hergestellt. Der knorpelige Anteil des Implantats wird letztlich genau auf das Gelenksniveau eingebracht.

Zusammenfassend sei der Vorteil der vorliegende Erfindung nochmals herausgestrichen:

Ein wesentlicher Aspekt der Erfindung liegt darin, dass durch das neue Verfahren erstmals ein autologer Knorpel für Transplantationszwecke hergestellt werden kann, der praktisch fast vollständig dem natürlichen entspricht. Wesentlich dabei ist, dass das Transplantat schon beim Einbau möglichst die selbe Form hat wie der natürliche Gelenksbelag. In der Natur ist der Knorpel mit dem darunterliegenden Knochen fest verbunden bzw. verzahnt. Eine ähnliche Verzahnung gelingt mittels der neuen Methode. Es wird ein Transplantat erhalten, das bereits beim Einbau ein natürliches Verhältnis zwischen Knochen-Knorpel-Anteil aufweist. Das neue Im- bzw. Transplantat ist an der Gelenksoberfläche knorpelig weich und geht in die Tiefe fließend in knochenhartes Material über - wie dies In der natürlichen Umgebung der Fall ist. Dieser fließende Übergang kann durch den unterschiedlich starken Grad der Demineralisierung erzielt werden und der wieder durch das unterschiedlich starke Angreifen der Salzsäure. Dies, ebenso wie das unterschiedlich starke Eindringen der Zellsuspension, wird durch die beschriebene Verjüngung der Bohrkanäle erreicht.

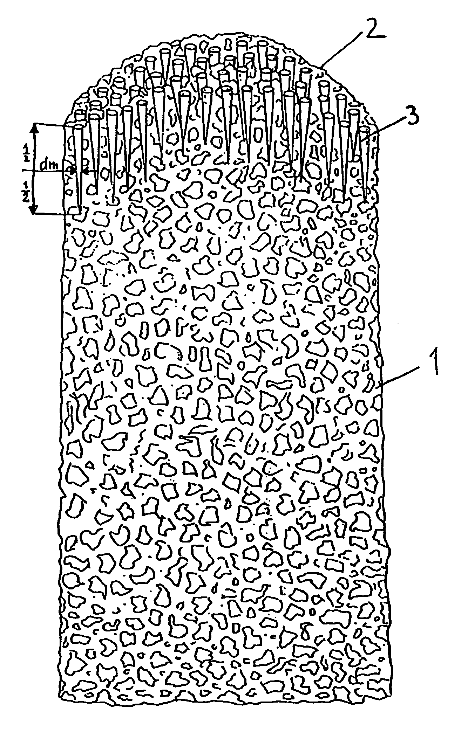

## Patentansprüche

1. Implantat *oder* Implantatkörper für die Regeneration von Gelenksdefekten im menschlichen oder tierischen Körper, welches bzw. welcher mit einem Trägerkörper (1) aus einem körperverträglichen, Poren- bzw. Schwammstruktur aufweisenden Material *oder* aus einem spongiösen Knochen menschlicher oder tierischer Herkunft, gebildet ist und zumindest teilweise mit einer Suspension von Gewebezellen tränkbar bzw. getränkt ist, **dadurch gekennzeichnet, dass** das Implantat *oder* der Implantatkörper mit einem Trägerkörper (1) mit zumindest von einem Teilbereich seiner Oberfläche (2) ausgehenden, innerhalb desselben endenden Kanälen (3) für die Infiltration der jeweils für die Regeneration vorgesehenen Gewebs-Zellsuspension versehen ist.

2. Implantat nach Anspruch 1, **dadurch gekennzeichnet, dass** der Trägerkörper mit einer Knorpel(gewebs)-Zellen-Suspension getränkt ist.

3. Implantat nach Anspruch 1 oder 2, **dadurch gekennzeichnet, dass** die Infiltrier-Kanäle (3) hohl-zylindrische Form und einen Durchmesser im Bereich von 300 bis 500 µm aufweisen, oder aber bevorzugter Weise von der Oberfläche (2) des Trägerkörpers (1) ausgehend, in Richtung zum Trägerkörper-Inneren hin sich verjüngende, spitzkonus- *oder* spitz-kegel- *oder* spitz-kegelstumpf-artige Form und jeweils mittig einen Durchmesser (dm) von 200 bis 500 µm aufweisen.

4. Implantat nach einem der Ansprüche 1 bis 3, **dadurch gekennzeichnet, dass** die Tiefe der Infiltrier-Kanäle (3) das 3- bis 10-Fache, insbesondere das 5- bis 10-Fache, ihres Durchmessers *oder* Mitten-Durchmessers (dm) beträgt

5. Implantat nach einem der Ansprüche 1 bis 4, **dadurch gekennzeichnet, dass** der Trägerkörper (1) Zylinder-Form aufweist, wobei zumindest von dessen Grund- und/oder Deckfläche (2) die Infiltrier-Kanäle (3) ausgehen.

6. Implantat nach einem der Ansprüche 1 bis 5, **dadurch gekennzeichnet, dass** jene Grund- *oder* Deckfläche (2) des zylinderförmigen Trägerkörpers (1), von welcher die Infiltrier-Kanäle (3) ausgehen, konvex gekrümmt ausgebildet ist.

7. Implantat nach einem der Ansprüche 1 bis 6, **dadurch gekennzeichnet, dass** der mit der Knorpelzellen-Suspension getränkte Bereich des Trägerkörpers (1) - für den Fall, dass er aus Knochenmaterial gebildet ist - zumindest teilweise demineralisiert ist.

8. Verfahren zur Herstellung eines mit gezüchteten Knorpelzellen zumindest teilweise getränkten Implantates *oder* Implantatkörpers für die Regeneration von Gelenksdefekten im menschlichen oder tierischen Körper nach einem der Ansprüche 1 bis 7, **dadurch gekennzeichnet, dass** in den Trägerkörper (1) von zumindest von einem Teilbereich seiner Oberfläche ausgehend, Infiltrationskanäle (3) eingebracht *oder* eingetieft werden, wonach der Trägerkörper (1) zumindest mit eingebracht *oder* eingetieft werden, wonach der Trägerkörper (1) zumindest mit diesem Teilbereich in die jeweils auszusiedelnde Zellen, vorzugsweise gezüchtete Knorpelzellen, enthaltende Suspension eingebracht *oder* eingetaucht wird.

9. Verfahren nach Anspruch 8, **dadurch gekennzeichnet, dass** in den Trägerkörper (1) mittels mechanischen Bohrens, Laserstrahl oder Druck-Wasserstrahl Infiltrationskanäle (3) eingebracht oder eingetieft werden, wonach der Trägerkörper (1) zumindest mit diesem Teilbereich in die gezüchtete Knorpelzellen enthaltende Suspension eingebracht oder eingetaucht wird, wobei es bevorzugt ist, wenn der Trägerkörper (1) nach dem Einbringen in die Knorpelzellen-Suspension mit einem unterhalb des Umgebungsdruckes liegenden Druck bzw. mit Vakuum beaufschlagt wird.

10. Verfahren nach Anspruch 8 *oder* 9, **dadurch gekennzeichnet, dass** zumindest der mit der Zellen- *oder* Knorpelzellen-Suspension zu tränkende Bereich des Trägerkörpers (1) vor dem Tränken einem Reinigungs- und/oder DemineralisierungsProzess unterzogen wird, wobei bevorzugterweise zumindest der zu tränkende Bereich des Trägerkörpers (1) in ein säure-hältiges Demineralisierungs-Medium, insbesondere *in* 0,5N Salzsäure, eingebracht wird und mit einem unterhalb des Umgebungsdrucks liegenden Druck oder mit Vakuum beaufschlagt wird, wonach das Demineralisierungs-Medium und die sich dann darin befindlichen, durch die Demineralisierung aus dem Trägerkörpermaterial herausgelösten Salze durch Spülen entfernt werden.

11. Verfahren nach *einem* der Ansprüche 8 *bis* 10, **dadurch gekennzeichnet, dass** zumindest der zu tränkende Bereich des Trägerkörpers (1) vor dem Tränken mit der Zellen- *oder* Knorpelzellen-Suspension mit einem die Bildung der Zellen *oder* Knorpelzellen begünstigenden Nährmedium, beispielsweise Hyaluronsäure oder Kollagen, imprägniert wird, oder dass das Tränken des Trägerkörpers mit einem Gemisch aus einer Zellen- *oder* Knorpelzellen-Suspension und aus einem die Bildung der Zellen *oder* Knorpelzellen begünstigenden Medium, beispielsweise Hyaluronsäure oder Kollagen, erfolgt.

## Claims

1. Implant or implant body for regenerating joint deficiencies in a human or animal body, which is formed by a carrier body (1) of a material that is acceptable to the body and has a porous or spongy structure, or is formed by a spongy bone of human or animal origin, and which is able to be soaked or is soaked at least in part with a suspension of tissue cells, **characterised in that** the implant or implant body is provided with a carrier body (1) comprising with channels (3) for infiltrating the respective tissue cell suspension, respectively provided for regeneration, which start from at least a partial area of its surface (2) and end in its interior.

2. Implant according to claim 1, **characterised in that** said carrier body is soaked with a cartilage (tissue) cell suspension.

3. Implant according to claim 1 or 2, **characterised in that** the infiltration channels (3) have a hollow cylindrical shape and a diameter within the range of 300 to 500 µm, or, preferably, starting from said surface (2) of said carrier body (1) are tapering in direction towards the interior of the carrier body, thus having a pointedly conical or a pointedly tapered or a pointed frustum shape have a diameter (dm) of 200 to 500 µm in the centre.

4. Implant according to any of claims 1 to 3, **characterised in that** the depth of said infiltration channels (3) is the 3-fold up to the 10-fold, particularly the 5-fold up to the 10-fold, of their diameter or central diameter (dm).

5. Implant according to any of claims 1 to 4, **characterised in that** said carrier body (1) has a cylindrical shape, said infiltration channels (3) starting from its basic and/or cover surface (2).

6. Implant according to any of claims 1 to 5, **characterised in that** that basic or cover surface (2) of the cylindrical carrier body (1), from which the infiltration channels (3) start, is curved in a convex manner.

7. Implant according to any of claims 1 to 6, **characterised in that** that area of the carrier body (1), which is soaked with a cartilage cell suspension, and in case it is formed of bone material, is demineralised at least in part.

8. A method for producing an implant or an implant body being at least partially soaked with cultured cartilage cells for regenerating joint deficiencies in a human or animal body according to any of claims 1 to 7, **characterised in that** infiltration channels (3) are worked or deepened into said carrier body (1), starting from at least a partial area of its surface, after which said carrier body (1) and at least this partial area is inserted or submerged into a suspension containing respective cells to be resettled, preferably cultured cartilage cells.

9. Method according to claim 8, **characterised in that** infiltration channels (3) are worked or deepened into said carrier body (1) by boring mechanically, by a laser beam or by a pressurised water jet, after which said carrier body (1) and at least this partial area is inserted or submerged into a suspension containing said cultured cartilage cells, wherein it is preferred, if said carrier body (1) is biased by a pressure below ambient pressure or by a vacuum after insertion into said cartilage cell suspension.

10. Method according to claim 8 or 9, **characterised in that** at least that area of said carrier body (1), which has to be soaked with said cell or cartilage cell suspension, is subjected to a purifying or demineralising procedure prior to soaking, wherein preferably at least the area to be soaked of said carrier body (1) is inserted into an acid containing demineralising medium, particularly into 0.5N hydrochloric acid, and is biased by a pressure below ambient pressure or by a vacuum, after which said demineralising medium and salts, which are then therein and have been extracted from the material of said carrier body by demineralisation, are removed by rinsing.

11. Method according to any of claims 8 to 10, **characterised in that** at least that area of said carrier body (1), which is to be soaked, is impregnated prior to soaking with said cell or cartilage cell suspension with a nutrient medium, which favours the formation of cells or cartilage cells, for example hyaluronic acid or collagen, or that soaking of said carrier body is done with a mixture of said cell or cartilage cell suspension with a medium, which favours the formation of cells or cartilage cells, for example hyaluronic acid or collagen.

## Revendications

1. Implant ou corps d'implant pour régénérer des défauts articulaires du corps humain ou animal, qui est formé avec un corps porteur (1) d'un matériau compatible au corps et comprend une structure poreuse ou spongieuse, ou comprend un os spongieux d'origine humain ou animal et peut être ou est imprégné au moins partiellement d'une suspension des cellules de tissu, **caractérisé en ce, que** l'implant ou le corps d'implant est pourvu d'un corps porteur (1) comprenant des canaux (3), qui partent au moins d'une aire partielle de sa surface (2) et terminent dans l'intérieur de ceci pour l'infiltration de la suspension des cellules de tissu prévue respectivement pour la régénération.

2. Implant selon la revendication 1, **caractérisé en ce, que** le corps porteur est imprégné d'un suspension de cellules (de tissu) de cartilage.

3. Implant selon la revendication 1 ou 2, **caractérisé en ce, que** les canaux d'infiltration (3) ont une forme creuse cylindrique et un diamètre dans la domaine de 300 à 500 µm, ou, partant de la surface (2) du corps porteur (1), ils ont de préférence une forme se réduisant en direction vers l'intérieur du corps porteur d'un cône pointu ou une forme conique pointue ou tronconique pointue et un diamètre (dm) au centre respectif de 200 à 500 µm.

4. Implant selon une quelconque des revendications 1 à 3, **caractérisé en ce, que** la profondeur des canaux d'infiltration (3) est 3 fois à 10 fois, particulièrement 5 fois à 10 fois son diamètre ou diamètre au centre (dm).

5. Implant selon une quelconque des revendications 1 à 4, **caractérisé en ce, que** le corps porteur (1) a une forme cylindrique, des canaux d'infiltration (3) partant au moins de sa surface de base et/ou la surface supérieure (2).

6. Implant selon une quelconque des revendications 1 à 5, **caractérisé en ce, que** celle surface de base ou supérieure (2) du corps porteur cylindrique (1), dont les canaux d'infiltration (3) partent, sont courbés d'une façon convexe.

7. Implant selon une quelconque des revendications 1 à 6, **caractérisé en ce, que** cette aire du corps porteur (1), qui est imprégnée avec la suspension de cellules de cartilage, en cas qu'il est formé d'un matériau osseux, est déminéralisée au moins partiellement.

8. Procédé de production d'un implant ou d'un corps d'implant au moins partiellement imprégné avec des cellules de cartilage cultivées pour régénérer des défauts articulaires du corps humain ou animal, selon une quelconque des revendications 1 à 7, **caractérisé en ce, que** des canaux d'infiltration (3) sont introduits ou approfondis dans le corps porteur (1) à partir d'au moins une aire partielle de sa surface, après quoi le corps porteur (1) est introduit ou immergé au moins avec cette aire partielle dans la suspension, qui contient des cellules respectives à transplanter, de préférence des cellules cultivées de cartilage.

9. Procédé selon la revendication 8, **caractérisé en ce, que** les canaux d'infiltration (3) sont introduits ou approfondis dans le corps porteur (1) au moyen d'un alésage mécanique, d'un rayon de laser ou d'un jet d'eau sous pression, après quoi le corps porteur (1) est introduit ou immergé au moins avec cette aire partielle dans la suspension, qui contient des cellules cultivées de cartilage, dans lequel il est préféré, si le corps porteur (1), après l'introduction de la suspension de cellules de cartilage, est soumis à une pression au dessous de la pression ambiante ou à un vacuum.

10. Procédé selon la revendication 8 ou 9, **caractérisé en ce, qu**'au moins cette aire du corps porteur (1), qui est à imprégner avec la suspension de cellules ou de cellules de cartilage, avant de l'imprégnation, est soumis d'une procédure de purification et/ou de déminéralisation, dans lequel au moins cette aire du corps porteur (1), qui est à imprégner, est introduite dans un milieu de déminéralisation contenant d'acide, particulièrement dans 0,5N d'acide chlorhydrique, et est soumis à une pression au dessous de la pression ambiante ou à un vacuum, après quoi on élimine le milieu de déminéralisation et les sels y étant et extraits du matériau du corps porteur par la déminéralisation par un rinçage.

11. Procédé selon une quelconque des revendications 8 à 10, **caractérisé en ce, qu**'au moins cette aire du corps porteur (1), qui est à imprégner, avant de l'imprégnation avec la suspension de cellules ou de cellules de cartilage, est imprégnée avec un milieu nutritif, qui favorise la formation des cellules ou des cellules de cartilage, par exemple avec l'acide hyaluronique ou avec du collagène, ou que l'imprégnation du corps porteur est réalisée avec un mélange d'une suspension de cellules ou de cellules de cartilage et un milieu nutritif, qui favorise la formation des cellules ou des cellules de cartilage, par exemple de l'acide hyaluronique ou du collagène.
